# EUROPEAN PATENT SPECIFICATION

(11) **EP 4 374 466 B1**
(45) Date of publication and mention of the grant of the patent: **19.11.2025**
(21) Application number: 22755141.3
(22) Date of filing: 22.07.2022
(51) Int. Cl.: A61N 1/05, A61N 1/372

(54) **DEVICE FOR CONTINUOUS INSERTION-DEPTH MONITORING FOR IMPLANTABLE LEAD**
VORRICHTUNG FÜR KONTINUIERLICHE ÜBERWACHUNG DER EINFÜHRUNGSTIEFE FÜR IMPLANTIERBARE LEITUNG
APPAREIL POUR SURVEILLANCE CONTINUE DE LA PROFONDEUR D'INSERTION D'UN FIL IMPLANTABLE

(30) Priority: 23.07.2021 EP 21187521
(43) Date of publication of application: 29.05.2024
(73) Proprietor: Universitair Ziekenhuis Antwerpen, 2650 Edegem (BE); Universiteit Antwerpen, 2000 Antwerpen (BE)
(72) Inventor: HUYBRECHTS, Wim, 2650 Edegem (BE)
(74) Representative: De Clercq & Partners
(86) International application number: PCT/EP2022/070578
(87) International publication number: WO 2023/002001

(56) References cited:
- WO-A1-2010/036313
- WO-A1-2022/066467
- US-A1- 2009 276 003
- US-A1- 2011 160 824
- US-A1- 2012 071 945

## Description

### Field of the invention

The present invention is in a field of implantable electrical-signal transmitting leads for cardiac applications, such as monitoring or stimulation. The lead may be connectable to a pacemaker, or a cardioverter-defibrillator (ICD) to treat a ventricular tachycardia or fibrillation.

### Background to the invention

A new form of physiological pacing has emerged recently in the field of cardiac pacing. To achieve natural, physiological activation of the heart during permanent cardiac pacing, an implantable lead is positioned on the bundle of His or in the left bundle branch area (LBBA) - part of the natural conduction system of the heart. This LBBA is reachable with an implantable lead by entering the ventricular septum (myocardium) from the right side, engaging the septum until reaching the subendocardial left side.

The implantable lead is inserted by axial rotation that drives a distal helical electrode into the myocardium. A problem in the art is how to continuously monitor insertion depth. Continuous monitoring has been found to be critical because a stepwise advancement entails a release of driving torque in the implantable lead between depth measurements e.g. by ECG-evaluation, medical imaging. Resumption of advancement requires that torque is built up again in the implantable lead, which can cause an overturning at the tip if there is too much torque applied, and puncture through the entire myocardium.

US 2011/160824 A1 discloses devices, systems, and methods for implanting and testing multi-conductor electrical leads are disclosed. An illustrative implant tool for use with an implantable lead includes a main body, a plurality of spring contact members, and a knob mechanism. The main body of the implant tool includes a distal clamping mechanism with an opening adapted to frictionally receive a terminal boot of the implantable lead. The spring contact members are configured to provide an interface for connecting electrical connectors from a Pacing System Analyzer (PSA) or other testing device to the terminal contacts on the implantable lead. A knob mechanism coupled to the main body can be actuated to engage a terminal pin of the implantable lead, allowing an implanting physician to engage a fixation helix into body tissue by rotating the mechanism.

WO 2022/066467 A1, being a prior art under Article 54(3) EPC, discloses an adapter configured to electrically connect a test device to an implantable medical lead comprises a rotational electrical coupling. The rotational electrical coupling is configured to electrically connect a lead electrical connector to an adapter electrical connector. The rotational electrical coupling comprises a first conductive component configured to be electrically connected to the lead electrical connector and rotatable with a proximal portion of the implantable medical lead, and a second conductive component electrically connected to the first conductive component and the adapter electrical connector. The second conductive component may be rotationally fixed. The first and second conductive components may comprise graphite or another soft metal.

The present invention provides a system according to claim 1.

### Summary of the invention

Provided herein is a system (100) as set out in claim 1.

The implantable lead (60) may be lumenless.

The implantable lead (60) may comprise a shaft body (66), whereby the distal helical electrode (64) is attached at distal end (10) of shaft body (66) in fixed axial rotation with the shaft body (66), and the lead proximal connector (62) is attached at proximal end (12) of shaft body (66) in fixed axial rotation with the shaft body (66).

The implantable lead (60) may be configured for transmission of torque between the distal helical electrode (64) and the lead proximal connector (62).

The implantable lead (60) may be configured for the transmission of the torque in the absence of a removeable stylet.

The implantable lead (60) distal helical electrode (64) may be configured for insertion into a left bundle branch area of the subject's myocardium, or into the His-bundle of the subject's myocardium.

The rotational interface (34) may be configured for continuous transmission of electrical signals between the distal helical electrode (64) and the adapter proximal connector (38), and to reduce or prevent twisting of an adapter proximal lead (37) connecting the proximal body (36b) to the adapter proximal connector (38) during the rotations of or induced in the distal body (36a).

The adapter proximal lead (37) may be adapted for transmission of:
electrical signals between the adapter proximal connector (38) and the proximal body (36b) via one or more electrically conductive cables within the adapter proximal lead (37), and
- electrical signals between the adapter proximal connector (38) and an anode connector (40) *via* one or more further electrically conductive cables within the adapter proximal lead (37).

The system (100) anode connector (40) may comprise a tissue fixation element for dismountable attachment to tissue of the subject.

The system (100) may further comprise the implantable lead (60).

The system (100) may further comprise the pacing spectrum analyser.

### Figure Legends

**FIG. 1** is a schematic view of an adapter as presently described.
**FIG. 2** is a schematic view of an adapter as presently described, with implantable lead.
**FIG. 3** is a schematic view of an adapter as presently described, with implantable lead and pacing spectrum analyser.
**FIG. 4** is an example of an adapter as presently described.
**FIG. 5** is an example of an adapter as presently described, provided with an anode clip.
**FIG. 6** is an example of an implantable lead connectable to an adapter as presently described.
**FIG. 7** is an example of an extension or adaptor cable as presently described.
**FIG. 8** is an example of pacing spectrum analyser connectable to an adapter as presently described.

### Detailed description

Before the present system is described, it is to be understood that this invention is not limited to particular systems described, but the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" or "distal to" and "proximal" or "proximal to" are used throughout the specification, and are terms generally understood in the field to mean towards (proximal) or away (distal) from the operator's side of system. Thus, "proximal" or "proximal to" means towards the operator's side and, therefore, away from the patient's side. Conversely, "distal" or "distal to" means towards the patient's side and, therefore, away from the operator's side. The system has a distal and proximal end and components of the system including the adapter, adapter distal connector, adapter distal lead, adapter proximal lead, adapter proximal connector, rotational interface have distal and proximal ends that correspond with distal and proximal ends of the system.

The term "rotation" as used herein typically refers to an axial rotation, unless otherwise specified.

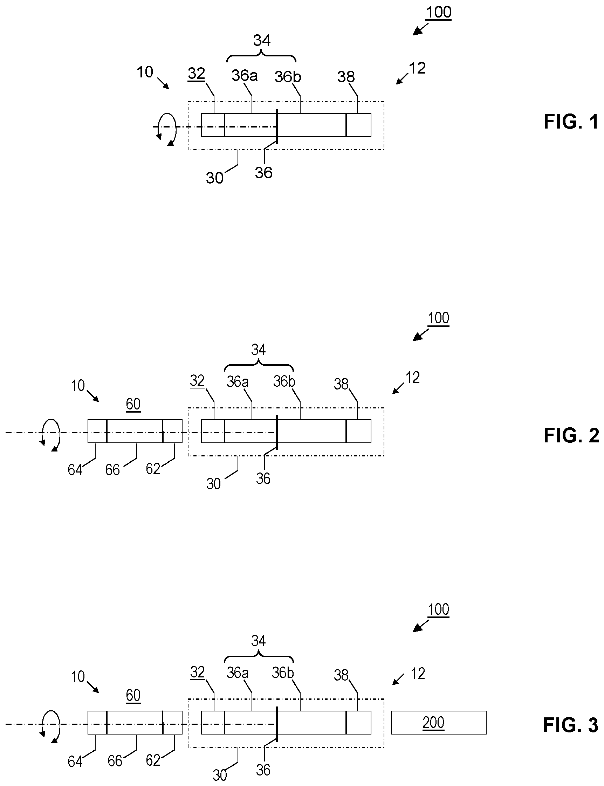

Provided herein is a system (100) for assisting regulated depth insertion of an implantable lead (60) into the heart muscle (myocardium) of a subject, wherein the implantable lead (60) is configured for transmission of electrical signals. An exemplary system is illustrated in **FIGs. 1 to 5****.**

The system (100) comprises an adapter (30) having a distal (10) end and a proximal (12) end. The adapter (100) comprises an adapter distal connector (32) configured for dismountable attachment to a lead proximal connector (62) of the implantable lead (60). The attachment is mechanical and electrical.

By mechanical attachment herein, it is meant that the respective parts are maintained attached to each other, whereby a mechanical force is required to attach and/or detach the parts, such as tension, torque, or compression, or other motion. The mechanical attachment may be achieved by means of a male-female fitting.

By electrical attachment herein, it is meant that the respective parts mutually attached to allow transmission of electrical current (e.g. electrical power, signals) across one or more electrically conducting cables in each of the parts. The electrical attachment may be achieved by one or more pairs of electrical contacts that come into alignment during mechanical attachment.

The adapter (100) further comprises a rotational interface (34), comprising a rotational joint (36) having a distal body (36a) rotationally attached to a proximal body (36b), the rotational interface (34) configured for conductance of electrical signals across the rotational joint (36).

The adapter (100) further comprises an adapter proximal connector (38) configured for dismountable mechanical and electrical connection to a pacing spectrum analyser (PSA) (200).

The adapter (30) is configured such that electrical signals are transmittable between a distal helical electrode (64) of the implantable lead (60) and the adapter proximal connector (38) during rotations of or induced in the distal body (36a).

The adapter (30) is further configured such that rotations of or induced in the distal body (36a) change the depth of the helical electrode (64) in the heart muscle.

The adapter (30) is further configured such that a rotation of the adapter proximal of the rotational joint (36) (e.g. proximal body (36b), adapter proximal connector (38)) is reduced or eliminated during the rotations of or induced in the distal body (36a).

The adapter (30) allows a continuous transmission of electrical signals between the helical electrode (64) and lead proximal connector (38). This means that insertion depth inside the heart muscle can be continuously monitored, based on continuous readings of electrical signals. During insertion, torque that rotates the helical electrode to advance or withdraw it, needs to be built up in the implantable lead (60).

Without the adapter, leads entering the PSA (200) would twist, and because multiple complete turns are required, the twisting creates a back torque that sets up resistance to further turning. To avoid this, the implantable lead (60) would need to be connected and disconnected between rotations. This creates a discontinuity in readings, and also requires that torque is built up again to effect the desired insertion or withdrawal of the helical electrode (64). Depending on curves inside the blood-vessels/sheath used to reach the septum, this torque build-up can sometimes be released uncontrollably at the distal helical electrode, risking sudden advancement and hence perforation all the way through the muscle. The slip ring assembly allows infinite rotations and does not introduce signal noise during rotation / depth adjustment - there is a stable electrical connection. The present system allows improved control by maintaining torque, preventing over-shooting, and giving a continuous, reduced-noise feedback. A wider range of subjects of differing heart morphologies, can be treated more easily because depth control is equal across a full range of rotations.

The adapter (30) may be configured for monodirectional, or more-preferably bidirectional transmission of signals. The electrical signals transmittable between the distal helical electrode (64) and the adapter proximal connector (38) means the signals may be transmitted in a proximal (12) to distal (10) direction or in a distal (10) to proximal (12) direction. The adapter (30) may be configured for transmission of electrical signals in both proximal (12) to distal direction (10) and in a distal (10) to proximal (12) direction. The direction of transmission may depend on a mode of operation. For instance, for use with a pacing spectrum analyser, the signals are bi-directional.

The adaptor distal connector (32) may be configured to mechanically attach in fixed axial rotational relation with respect to the lead proximal connector (62). The adaptor distal connector (32) and lead proximal connector (62) may be axially aligned when mechanically and electrically connected.

The distal connector (32) may be attached to the distal body (36a) via an adapter distal lead (33). Adapter distal lead (33) may be flexible e.g. bendable/foldable along its axis. Adapter distal lead (33) may be able to adapted for transmission of electrical signals between the adapter distal connector (32) and the distal body (36a) *via* one or more electrically conductive cables within the adapter distal lead (33).

The adapter distal lead (33) may be adapted to transmit torque. The torque transmission between the distal (10) and proximal (20) end of the adapter distal lead (33) may have no lag or backlash, or a limited degree of lag or backlash. Where there is a limited degree of lag or backlash, a small initial rotation of proximal end (20) is required before the distal end (10) rotates synchronously. Where lag is present, it may limited to a value between 1 and 90°, between 1 and 45°, preferably between 1 and 15°.

The adapter distal lead (33) may be disposed with at least one cable fray protector to prevent or reduce fraying of the adapter distal lead (33) where it attaches to the distal body (36a) or to the adaptor distal connector (32). An example of a cable fray protector is a flexible sheath. The flexible sheath may be made from a stretchable material (e.g. rubberised), or a non-stretchable material disposed with flexibility slots.

The adapter distal connector (32) may be attached in fixed rotational relation to the distal body (36a). An attachment in fixed rotational relation means that axial rotations of the attached parts are synchronised or have the aforementioned limited degree of lag or backlash. The adapter distal lead (33) is preferably axially non-compliant. The adapter distal lead (33) may be axially compliant and biased in non-twisted state (e.g. like a torsion spring). The axial compliance is limited to the aforementioned lag value. To achieve attachment in fixed rotational relation to the distal body (36a), the adapter distal connector (32) may comprise any fitting configured to rotationally fix the respective parts, such as a threaded-fitting, a clamp, a rotation limiter, or a combination of these.

Adapter distal connector (32) typically comprises a female type fitting for connection to the lead proximal connector (62) of the implantable lead (60).

The adapter distal connector (32) may be configured for connection to a standard connector such as a IS-1 connector (pacing lead), a 3/4 mm header (pacing lead), a 5/6 mm header (pacing lead), or DF-4 (ICD lead). The adapter distal connector (32) may be a universal connector configured for connection to at least two (preferably all) different standard connectors such as a IS-1 connector (pacing lead), a 3/4 mm header (pacing lead), a 5/6 mm header (pacing lead), or DF-4 (ICD lead).

The adapter distal connector (32) may be configured for connection to a non-standard connector such as an adapted IS-1 connector (pacing lead), an adapted 3/4 mm header (pacing lead), an adapted 5/6 mm header (pacing lead), or an adapted DF-4 (ICD lead).

Rotational Interface (34) comprise one or more slip ring assemblies. Electrical signals are conducted across the rotational joint (36) via the one or more slip ring assemblies during infinite rotations for the rotational joint (36). A slip ring assembly typically comprises a conductive slip ring disposed in fixed rotational relation with one of the distal body (36a) or proximal body (36b) and co-axial with the axis of the rotational joint (36), and a brush or wiper in electrical contact with the conductive ring and disposed in fixed rotational relation with the other of the proximal body (36b) or the distal body (36a). Force is usually applied to the brush or wiper to maintain continuous contact with the slip ring, using for instance a spring. The rotational joint (36) is preferably a 1 degree of freedom (DOF) joint. The distal body (36a) is preferably rotationally attached to the proximal body (36b) by a joint having only 1 degree of freedom of rotation. The rotational interface may be enclosed in a protective housing.

The adapter proximal connector (38) is configured for electrical attachment to the PSA (200), in particular to the PSA connector (202). The attachment may be repeatable. The attachment may be direct or indirect.

A direct attachment is where the adapter proximal connector (38) is in direct mechanical (and electrical) contact with the PSA (200), in particular with the PSA connector (202). There are no intervening extension or adapter cables. The adapter proximal connector (38) may be configured for mechanical attachment to the PSA (200), in particular to the PSA connector (202). The adaptor distal connector (32) may be configured to mechanically attach in fixed axial rotational relation with respect to the PSA connector (202).

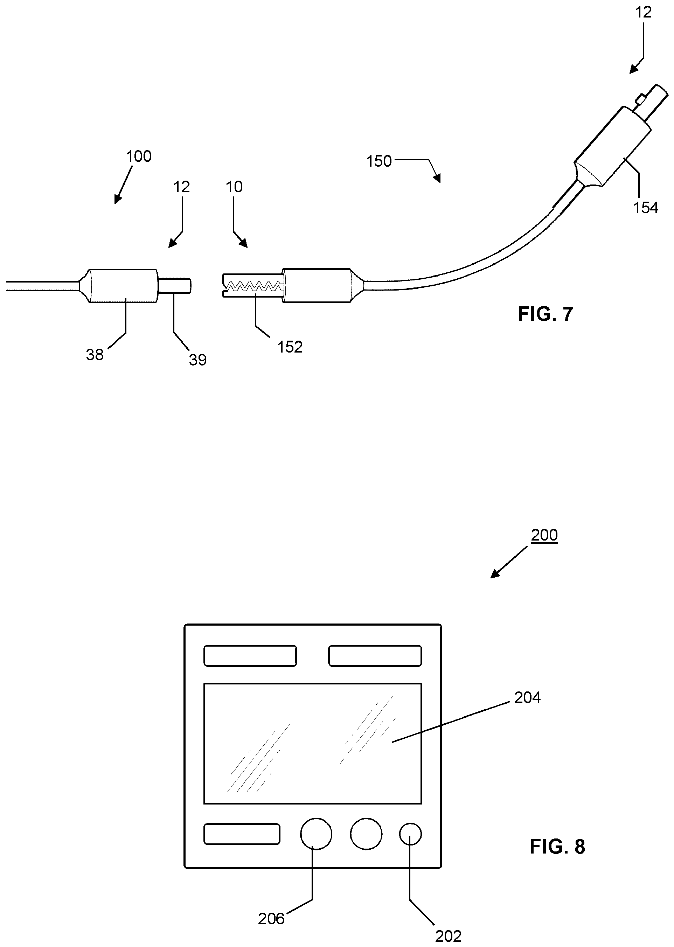

An indirect attachment is where the adapter proximal connector (38) is in electrical (and mechanical) contact with the PSA connector (202) *via* one or more dismountable intervening extension or adapter cables (150). An exemplary intervening extension or adapter cable is shown in **FIG. 7****.**

One intervening extension or adapter cable (150) may be configured at one (proximal (12)) end for direct (mechanical and electrical) contact with the PSA (200), in particular with the PSA connector (202). The attachment may be *via* a universal connector to fit different types PSA connector (202). The attachment may be *via* a specific connector (154) to fit a specific PSA connector (202). The other (distal (10)) end of the extension or adapter cable (150) may be configured for direct (mechanical and electrical) contact with the adapter proximal connector (38). The attachment may be *via* a complementary connector or *via* clamping connector (*e.g.* crocodile clip (152)).

Where there are two or more dismountable intervening extension or adapter cables, they may be configured for dismountable connection in a daisy chain, one end of the daisy chain configured for direct (mechanical and electrical) contact with the PSA (200), in particular with the PSA connector (202), and the other end configured for direct (mechanical and electrical) contact with the adapter proximal connector (38).

The adapter proximal connector (38) may comprise at least one electrically conductive contact (39) configured for mechanical and electrical attachment to a crocodile clip (152) of an intervening extension or adapter cable (150). The at least one electrically conductive contact may contain serrations or indentations; these improve mechanical attachment.

The adapter proximal connector (38) may be attached to the proximal body (36b) via an adapter proximal lead (37). The adapter proximal lead (37) may be flexible e.g. bendable/foldable along its axis. The adapter proximal lead (37) may be able to transmit torque e.g. does not become twisted upon rotation. The adapter proximal lead (37) not may be able to transmit torque e.g. becomes twisted upon rotation.

The adapter proximal lead (37) may be adapted for transmission of electrical signals between the adapter proximal connector (38) and the proximal body (36b) via one or more (cathode) electrically conductive cables disposed within the adapter proximal lead (37).

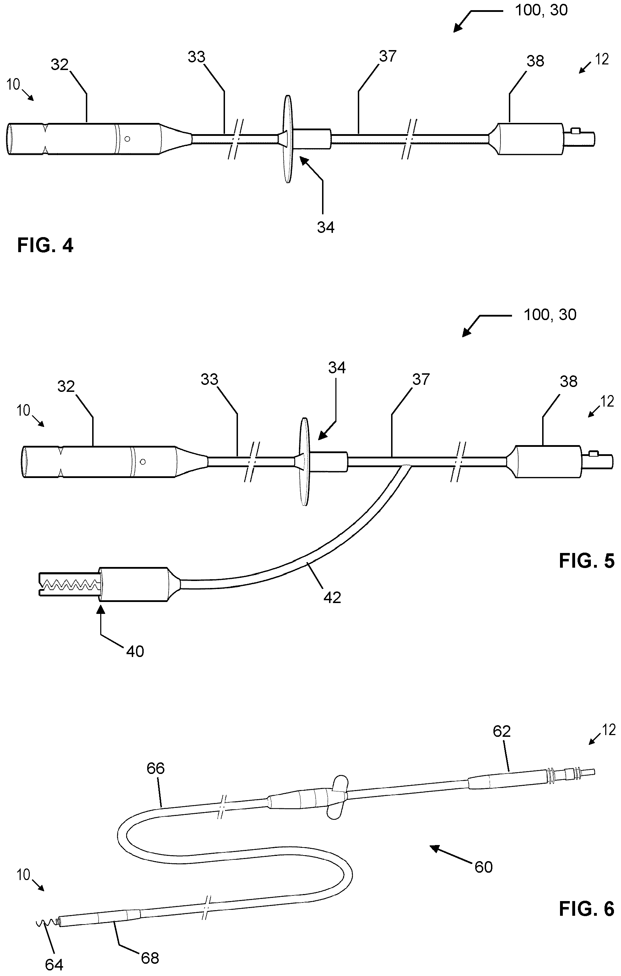

The adapter proximal lead (37) may be further adapted for transmission of electrical signals between the adapter proximal connector (38) and an anode connector (40) via one or more further (anode) electrically conductive cables (42). An example is shown in **FIG. 5****.** The one or more further (anode) electrically conductive cables (42) may be disposed at least partially within the adapter proximal lead (37). The anode connector (40) may comprises a tissue fixation element (*e.g.* spring clip) for dismountable attachment to tissue of the subject. The (anode) electrically conductive cables (42) may or may not be integrated into the adapter proximal lead (37).

The adapter proximal lead (37) may be disposed with at least one cable fray protector to prevent or reduce fraying of the adapter proximal lead (37) where it attaches to the proximal body (36b) or to the adapter proximal connector (38). An example of a cable fray protector is a flexible sheath. The flexible sheath may be made from a stretchable material (*e.g.* rubberised), or a non-stretchable material disposed with flexibility slots.

The adapter proximal connector (38) typically comprises a male type fitting for mechanical and electrical connection to the PSA connector (202).

Rotations (axial) of the adapter proximal lead (37) and of the adapter proximal connector (38) are prevented or reduced by the rotational interface (34). Full rotations of the (axial) of the adapter proximal lead (37) and adapter proximal connector (38) are prevented by the rotational interface (34), more specifically by the rotational joint (36).

The implantable lead (60) has a distal (10) end and a proximal (12) end. An exemplary implantable lead (60) is shown in **FIG. 6****.** The implantable lead (60) may be lumenless; in other words, it has no lumen, for instance, no lumen for a stiffening stylet.

The implantable lead (60) comprises shaft body (66), distal helical electrode (64), lead proximal connector (62). The distal helical electrode (64) is attached at distal end (10) of shaft body (66) in fixed axial rotation with the shaft body (66). The lead proximal connector (62) is attached at proximal end (12) of shaft body (66) in fixed axial rotation with the shaft body (66). The shaft body (66) may be flexible e.g. bendable/foldable along its axis.

The shaft body (66) may be adapted to transmit torque. The torque transmission between the distal (10) and proximal (20) end of the shaft body (66) may have no lag nor backlash, or a limited degree of lag or backlash. Where there is a limited degree of lag or backlash, a small initial rotation of proximal end (20) is required before the distal end (10) rotates synchronously. Where lag is present, it may limited to between 1 and 180°, between 1 and 90°, preferably between 1 and 45°.

The distal helical electrode (64) may be attached in fixed or constricted rotational relation to the lead proximal connector (62). An attachment in fixed rotational relation means that axial rotations of the attached parts are synchronised or have the aforementioned limited degree of lag or backlash. The shaft body (66) is preferably axially non-compliant. The shaft body (66) may be axially compliant and biased in non-twisted state (e.g. like a torsion spring). The axial compliance is limited to the aforementioned lag value.

The distal helical electrode (64) is configured for implantation into heart tissue by rotation of the shaft body (66) and/or lead proximal connector (62) and/or distal body (36a). The distal helical electrode (64) comprises a stiff wire having a helical or spiral path that extends in a longitudinal direction. Rotation of the distal helical electrode (64) within the muscle causes advancement or withdrawal of the distal helical electrode (64) depending on the direction of rotation (clockwise or anticlockwise). The tip of the distal helical electrode (64) may be sharpened to allow piercing of the muscle wall. Distal helical electrode (64) is configured for conductance of electrical signals. Typically, it is at least partially metallic; it may be made at least partially from for instance, platinum, or platinum-iridium alloy, or gold, or silver, or tin, or is gold plated, or silver plated, or tin plated. Distal helical electrode (64) may be a cathode.

The implantable lead (60) may further comprise one or more shaft (ring) electrodes (68) at the distal end for conductance of electrical signals. A pacing lead typically has one shaft electrode (and a corresponding IS-1 lead proximal connector (62) such as an IS-1 connector, a 3/4 mm header, or a 5/6 mm header). A defibrillation lead may have three shaft electrodes (and a corresponding DF-4 lead proximal connector (62)). Typically, the shaft electrode (68) is at least partially metallic; it may be made at least partially from for instance, platinum, or platinum- iridium alloy, or gold, or silver, or tin, or is gold plated, or silver plated, or tin plated. The shaft electrode (68) may be an anode. It is not usually employed during insertion.

Electrical signals are transmitted from the distal electrode(s) (helical electrode (64) and/or shaft electrode (68)), along one or more electrically conductive cables within the shaft body (66) to the lead proximal connector (62).

The implantable lead (60) may be a pacing lead. A pacing lead transmits electrical signals between its distal electrode(s) and a PSA and/or pacemaker in order to induce rhythmic contraction. An example of a pacing lead is a Medtronic SelectSecure 3830 pacing lead. The implantable lead (60) may be a cardioverter-defibrillator (ICD) lead. An ICD lead transmits electrical signals between its distal electrode(s) (helical electrode (64) and/or shaft electrode (68)), and a PSA and/or implantable cardioverter-defibrillator (ICD) in order treat a cardiac tachyarrhythmia.

The implantable lead (60) lead proximal connector (62) may be a standard lead connector such as IS-1 (pacing lead), 3/4 mm header (pacing lead), 5/6 mm header (pacing lead), or DF4 (ICD lead). The implantable lead (60) lead proximal connector (62) may be a non-standard lead connector. It may, for instance, be a modification of a standard lead connector.

One or more electrical signal parameters may be continuously monitored during insertion of the distal helical electrode (64). Relevant electrical signal parameters may include signal amplitude, impedance (over the system), and pacing threshold (minimum energy needed for stimulation).

The monitoring of the impedance parameter prevents perforation of the cardiac muscle. The impedance gradually drops while the electrode advances within a muscle, and suddenly drops steeply if it perforates through to the other side of the muscle. When an impedance drop is seen of 100-150 Ohms compared to the initial site the depth is considered satisfactory.

The ECG QRS-morphology during insertion (Rsr' or qR pattern) also gives an indication of depths inside the muscle. This morphology changes dependent on the position of the lead. In this way, the left position can be identified more accurately. The QRS morphology is also an indication of the ECG-pattern. Fixation beats are heart beats provoked by cardiac tissue irritation caused by piercing of the muscle and insertion of the distal helical electrode. Fixation beats have a different QRS-morphology depending on the depth inside the muscle; the depth can hence be determined from the fixation beats QRS-morphology. Pacing during inserting the lead inside the muscle, shows also a QRS-morphology change, according to the helical electrode depth; this may be more reliable as every pacing-pulse produces a QRS, whereas fixation beats are sometimes very scarce or even absent, rendering it a less reliable way to monitor lead depth.

The pacing spectrum analyser, PSA, (200) is configured to generate a real-time signal display, usually graphical, reflecting electrical signals measured by the helical electrode (32) as a function of time. An exemplary pacing spectrum analyser, PSA, (200) is shown in **FIG**. **8**. From the electrical signals, an optimum depth of the helical electrode (32) can be determined. Relevant parameters of the electrical signals that may be displayed by the PSA may include signal amplitude, impedance (over the system), pacing threshold (minimum energy needed to stimulation), QRS-morphology can be identified more accurately. The QRS morphology may give a partial indication of the ECG-pattern.

The pacing spectrum analyser (200) comprises a PSA connector (202) configured for dismountable mechanical and electrical connection to the adapter proximal connector (38), or to the universal connector or a specific connector (154) of the intervening extension or adapter cable (150). Typically, the PSA takes the incoming electrical signals from the distal helical electrode (64) and anode connector (40), amplifies it using an amplifier, digitises the amplified signal using an analogue to digital converter, processes the signal using a signal processor (e.g. to display correct scale and amplitude), and displays it on a screen (204).

The system (100) may further comprise one or more of:
- the implantable lead (60);
- pacing spectrum analyser (PSA) (200);
- tool for rotation of the lead (60) or lead proximal connector (62) or distal body (36a).

The distal helical electrode (64) is configured for insertion in to the myocardium.

The distal helical electrode (64) may be configured for insertion into the left bundle branch area of the subject's myocardium.

The distal helical electrode (64) may be configured for insertion into the His-bundle of the subject's myocardium.

## Claims

1. A system (100) for assisting regulated depth insertion of an implantable lead (60) into the heart muscle of a subject, wherein the lead (60) is configured for transmission of electrical signals, the system (100) comprising:
- an adapter (30) having a distal (10) end and a proximal (12) end comprising:
∘ an adapter distal connector (32) configured for dismountable mechanical and electrical attachment to a lead proximal connector (62) of the implantable lead (60), wherein the adaptor distal connector (32) is configured to mechanically attach in fixed rotational relation with respect to the lead proximal connector (62);
∘ a rotational interface (34), comprising a rotational joint (36) having a distal body (36a) rotationally attached to a proximal body (36b), the rotational interface (34) comprising one or more slip ring assemblies and configured for conductance of electrical signals across the rotational joint (36);
∘ an adapter proximal connector (38) configured for dismountable mechanical and electrical connection to a pacing spectrum analyser (200);
the adapter (30) being configured such that:
▪ electrical signals are transmittable between a distal helical electrode (64) of the implantable lead (60) and the adapter proximal connector (38) during rotations of or induced in the distal body (36a);
▪ the rotations of or induced in the distal body (36a) change the depth of the helical electrode (64) in the heart muscle;
▪ a rotation of the adapter proximal of the rotational joint is reduced or eliminated during the rotations of or induced in the distal body (36a).

2. The system (100) according to claim 1, further comprising the implantable lead (60).

3. The system (100) according to claim 2, wherein the implantable lead (60) comprises shaft body (66), whereby the distal helical electrode (64) is attached at distal end (10) of shaft body (66) in fixed axial rotation with the shaft body (66), and the lead proximal connector (62) is attached at proximal end (12) of shaft body (66) in fixed axial rotation with the shaft body (66).

4. The system (100) according to any one of claims 2 to 3, wherein the implantable lead (60) is configured for transmission of torque between the distal helical electrode (64) and the lead proximal connector (62).

5. The system (100) according to claim 4, wherein the implantable lead (60) is configured for the transmission of the torque in the absence of a removeable stylet.

6. The system (100) according to any one of claims 2 4 to 5, wherein the implantable lead (60) distal helical electrode (64) is configured for insertion into a left bundle branch area of the subject's myocardium, or into the His-bundle of the subject's myocardium.

7. The system (100) according to any one of claims 2 4 to 6, wherein the rotational interface (34) is configured for continuous transmission of electrical signals from the distal helical electrode (64) to the adapter proximal connector (38), and to reduce or prevent twisting of an adapter proximal lead (37) connecting the proximal body (36b) to the adapter proximal connector (38) during the rotations of or induced in the distal body (36a).

8. The system (100) according to claim 7, wherein the adapter proximal lead (37) is adapted for transmission of:
- electrical signals between the adapter proximal connector (38) and the proximal body (36b) via one or more electrically conductive cables within the adapter proximal lead (37), and
- electrical signals between the adapter proximal connector (38) and an anode connector (40) *via* one or more further electrically conductive cables within the adapter proximal lead (37).

9. The system (100) according to claim 8, wherein the anode connector (40) comprises a tissue fixation element for dismountable attachment to tissue of the subject.

10. The system (100) according to any one of claims 7 to 9, wherein the adapter proximal lead (37) is further adapted for transmission of electrical signals between the adapter proximal connector (38) and an anode connector (40) *via* one or more further anode electrically conductive cables (42).

11. The system (100) according to claim 2, wherein the implantable lead (60) is lumenless.

12. The system (100) according to any one of claims 1 to 11, further comprising the pacing spectrum analyser (200).

13. The system (100) according to any one of claims 1 to 12, wherein the distal connector (32) is attached to the distal body (36a) *via* an adapter distal lead (33).

## Patentansprüche

1. System (100) zum Unterstützen der Einführung einer implantierbaren Leitung (60) in eine geregelte Tiefe in den Herzmuskel eines Subjekts, wobei die Leitung (60) zur Übertragung elektrischer Signale ausgestaltet ist, wobei das System (100) umfasst:
- einen Adapter (30) mit einem distalen (10) Ende und einem proximalen (12) Ende, der Folgendes umfasst:
o einen distalen Adapterverbinder (32), der zur demontierbaren mechanischen und elektrischen Befestigung an einem proximalen Leitungsverbinder (62) der implantierbaren Leitung (60) ausgestaltet ist, wobei der distale Adapterverbinder (32) dazu ausgestaltet ist, in einer festen Drehbeziehung in Bezug auf den proximalen Leitungsverbinder (62) mechanisch angebracht zu werden;
o eine Drehschnittstelle (34), umfassend ein Drehgelenk (36) mit einem distalen Körper (36a), der drehbar an einem proximalen Körper (36b) angebracht ist, wobei die Drehschnittstelle (34) eine oder mehrere Schleifringanordnungen umfasst und für die Leitfähigkeit elektrischer Signale über das Drehgelenk (36) ausgestaltet ist;
o einen proximalen Adapterverbinder (38), der zur demontierbaren mechanischen und elektrischen Verbindung mit einem Schrittmacherspektrumanalysator (200) ausgestaltet ist;
wobei der Adapter (30) so ausgestaltet ist, dass:
▪ elektrische Signale zwischen einer distalen Spiralelektrode (64) der implantierbaren Leitung (60) und dem proximalen Adapterverbinder (38) während Drehungen des distalen Körpers (36a) oder darin induzierten Drehungen übertragbar sind;
▪ die Drehungen des distalen Körpers (36a) oder die darin induzierten Drehungen die Tiefe der Spiralelektrode (64) in dem Herzmuskel ändern;
▪ eine Drehung des Adapters proximal des Drehgelenks während der Drehungen des distalen Körpers (36a) oder der darin induzierten Drehungen reduziert oder eliminiert wird.

2. System (100) nach Anspruch 1, das ferner die implantierbare Leitung (60) umfasst.

3. System (100) nach Anspruch 2, wobei die implantierbare Leitung (60) Schaftkörper (66) umfasst, wobei die distale Spiralelektrode (64) am distalen Ende (10) des Schaftkörpers (66) in fester axialer Drehung mit dem Schaftkörper (66) befestigt ist, und der proximale Leitungsverbinder (62) am proximalen Ende (12) des Schaftkörpers (66) in fester axialer Drehung mit dem Schaftkörper (66) befestigt ist.

4. System (100) nach einem der Ansprüche 2 bis 3, wobei die implantierbare Leitung (60) zur Übertragung eines Drehmoments zwischen der distalen Spiralelektrode (64) und dem proximalen Anschlussverbinder (62) ausgestaltet ist.

5. System (100) nach Anspruch 4, wobei die implantierbare Leitung (60) für die Übertragung des Drehmoments in Abwesenheit eines entfernbaren Mandrins ausgestaltet ist.

6. System (100) nach einem der Ansprüche 2 bis 5, wobei die distale Spiralelektrode (64) der implantierbaren Leitung (60) zum Einführen in einen linken Tawara-Schenkelbereich des Myokards des Subjekts oder in das His-Bündel des Myokards des Subjekts ausgestaltet ist.

7. System (100) nach einem der Ansprüche 2 bis 6, wobei die Drehschnittstelle (34) ausgestaltet ist zur kontinuierlichen Übertragung elektrischer Signale von der distalen Spiralelektrode (64) zu dem proximalen Adapterverbinder (38) und zum Reduzieren oder Verhindern eines Verdrehens einer proximalen Adapterleitung (37), die den proximalen Körper (36b) mit dem proximalen Adapterverbinder (38) verbindet, während der Drehungen des distalen Körpers (36a) oder der darin induzierten Drehungen.

8. System (100) nach Anspruch 7, wobei die proximale Adapterleitung (37) zur Übertragung von Folgendem ausgestaltet ist:
- elektrischen Signalen zwischen dem proximalen Adapterverbinder (38) und dem proximalen Körper (36b) über ein oder mehrere elektrisch leitfähige Kabel innerhalb der proximalen Adapterleitung (37), und
- elektrischen Signalen zwischen dem proximalen Adapterverbinder (38) und einem Anodenverbinder (40) über ein oder mehrere weitere elektrisch leitfähige Kabel innerhalb der proximalen Adapterleitung (37).

9. System (100) nach Anspruch 8, wobei der Anodenverbinder (40) ein Gewebefixierungselement zur demontierbaren Befestigung an Gewebe des Subjekts umfasst.

10. System (100) nach einem der Ansprüche 7 bis 9, wobei die proximale Adapterleitung (37) ferner zur Übertragung elektrischer Signale zwischen dem proximalen Adapterverbinder (38) und einem Anodenverbinder (40) über ein oder mehrere weitere elektrisch leitfähige Anodenkabel (42) ausgestaltet ist.

11. System (100) nach Anspruch 2, wobei die implantierbare Leitung (60) lumenlos ist.

12. System (100) nach einem der Ansprüche 1 bis 11, ferner umfassend den Schrittmacherspektrumanalysator (200).

13. System (100) nach einem der Ansprüche 1 bis 12, wobei der distale Verbinder (32) über eine distale Adapterleitung (33) an dem distalen Körper (36a) befestigt ist.

## Revendications

1. Système (100) d'aide à l'insertion en profondeur régulée d'une sonde (60) implantable dans le muscle cardiaque d'un sujet, dans lequel la sonde (60) est conçue pour une transmission de signaux électriques, le système (100) comprenant :
- un adaptateur (30) ayant une extrémité distale (10) et une extrémité proximale (12) comprenant :
o un raccord distal d'adaptateur (32) conçu pour une liaison mécanique et électrique démontable à un raccord proximal de sonde (62) de la sonde (60) implantable, dans lequel le raccord distal d'adaptateur (32) est conçu pour une fixation mécanique dans une relation de rotation fixe par rapport au raccord proximal de sonde (62) ;
o une interface de rotation (34), comprenant un joint rotatif (36) ayant un corps distal (36a) attaché de manière rotative à un corps proximal (36b), l'interface de rotation (34) comprenant un ou plusieurs ensembles de bagues collectrices et étant conçue pour la conductance de signaux électriques de part et d'autre du joint rotatif (36) ;
o un raccord proximal d'adaptateur (38) conçu pour une liaison mécanique et électrique démontable à un analyseur de spectre de stimulation (200) ;
l'adaptateur (30) étant conçu de telle sorte que :
▪ des signaux électriques puissent être transmis entre une électrode hélicoïdale distale (64) de la sonde (60) implantable et le raccord proximal d'adaptateur (38) pendant des rotations du corps distal (36a) ou induites dans celui-ci ;
▪ les rotations du corps distal (36a) ou induites dans celui-ci modifient la profondeur de l'électrode hélicoïdale (64) dans le muscle cardiaque ;
▪ une rotation de l'adaptateur proximal du joint rotatif est réduite ou éliminée pendant les rotations du corps distal (36a) ou induites dans celui-ci.

2. Système (100) selon la revendication 1, comprenant en outre la sonde (60) implantable.

3. Système (100) selon la revendication 2, dans lequel la sonde (60) implantable comprend un corps d'arbre (66), moyennant quoi l'électrode hélicoïdale distale (64) est fixée au niveau de l'extrémité distale (10) du corps d'arbre (66) en rotation axiale fixe avec le corps d'arbre (66), et le raccord proximal de sonde (62) est fixé au niveau de l'extrémité proximale (12) du corps d'arbre (66) en rotation axiale fixe avec le corps d'arbre (66).

4. Système (100) selon l'une quelconque des revendications 2 à 3, dans lequel la sonde (60) implantable est conçue pour une transmission de couple entre l'électrode hélicoïdale distale (64) et le raccord proximal de sonde (62).

5. Système (100) selon la revendication 4, dans lequel la sonde (60) implantable est conçue pour la transmission du couple en l'absence d'un stylet amovible.

6. Système (100) selon l'une quelconque des revendications 2 à 5, dans lequel l'électrode hélicoïdale distale (64) de la sonde (60) implantable est conçue pour être insérée dans une zone de la branche gauche du faisceau de His du myocarde du sujet, ou dans le faisceau His du myocarde du sujet.

7. Système (100) selon l'une quelconque des revendications 2 à 6, dans lequel l'interface de rotation (34) est conçue pour une transmission continue de signaux électriques de l'électrode hélicoïdale distale (64) au raccord proximal d'adaptateur (38), et pour réduire ou empêcher une torsion d'une sonde proximale d'adaptateur (37) reliant le corps proximal (36b) au raccord proximal d'adaptateur (38) pendant les rotations du corps distal (36a) ou induites dans celui-ci.

8. Système (100) selon la revendication 7, dans lequel la sonde proximale d'adaptateur (37) est conçue pour une transmission de :
- signaux électriques entre le raccord proximal d'adaptateur (38) et le corps proximal (36b) par l'intermédiaire d'un ou de plusieurs câbles électroconducteurs à l'intérieur de la sonde proximale d'adaptateur (37), et
- signaux électriques entre le raccord proximal d'adaptateur (38) et un raccord d'anode (40) par l'intermédiaire d'un ou de plusieurs câbles électroconducteurs supplémentaires à l'intérieur de la sonde proximale d'adaptateur (37).

9. Système (100) selon la revendication 8, dans lequel le raccord d'anode (40) comprend un élément de fixation de tissu pour une fixation démontable au tissu du sujet.

10. Système (100) selon l'une quelconque des revendications 7 à 9, dans lequel la sonde proximale d'adaptateur (37) est en outre conçue pour une transmission de signaux électriques entre le raccord proximal d'adaptateur (38) et un raccord d'anode (40) par l'intermédiaire d'un ou de plusieurs câbles électroconducteurs (42) d'anode supplémentaires.

11. Système (100) selon la revendication 2, dans lequel la sonde (60) implantable est sans lumière.

12. Système (100) selon l'une quelconque des revendications 1 à 11, comprenant en outre l'analyseur de spectre de stimulation (200).

13. Système (100) selon l'une quelconque des revendications 1 à 12, dans lequel le raccord distal (32) est fixé au corps distal (36a) par l'intermédiaire d'une sonde distale d'adaptateur (33).
